# FASCICULE DE BREVET EUROPEEN

(11) **EP 1 769 788 B1**
(45) Date de publication et mention de la délivrance du brevet: **09.06.2010**
(21) Numéro de dépôt: 06120443.4
(22) Date de dépôt: 11.09.2006
(51) Int. Cl.: A61K 8/35, A61K 8/34, A61K 8/368, A61Q 5/00, A61Q 17/04

(54) **Procédé de traitement photoprotecteur des fibres kératiniques teintes artificiellement par application d'un mélange eau liquide/vapeur d'eau**
Lichtschutzverfahren zur Behandlung von gefärbten Keratinfasern durch Aufbringen von Wasser-Dampf-Mischungen
Process for the photoprotective treatment of artificially dyed keratin fibres by application of a liquid water/steam mixture

(30) Priorité: 29.09.2005 FR 0552956
(43) Date de publication de la demande: 04.04.2007
(73) Titulaire: L'Oréal, 75008 Paris (FR)
(72) Inventeur: Lalleman, Boris, 75015 Paris (FR)
(74) Mandataire: Miszputen, Laurent

(56) Documents cités:
- EP-A- 0 437 006
- EP-A- 0 659 396
- EP-A- 0 659 400
- EP-A- 0 662 314
- EP-A- 0 699 430
- EP-A- 1 118 319
- DE-A1- 10 051 773
- DATABASE CA [Online] CHEMICAL ABSTRACTS SERVICE, COLUMBUS, OHIO, US; 30 septembre 1995 (1995-09-30), GO, NAOHISA ET AL: "Hair treatment method without using a shower" XP002388984 extrait de STN Database accession no. 1995:823743 -& PATENT ABSTRACTS OF JAPAN vol. 1995, no. 10, 30 novembre 1995 (1995-11-30) & JP 07 187961 A (KAO CORP), 25 juillet 1995 (1995-07-25)

## Description

La présente invention a pour objet un procédé de traitement des fibres kératiniques teintes artificiellement par coloration directe ou par coloration d'oxydation, en particulier des cheveux humains, qui comprend l'application sur lesdites fibres kératiniques d'une composition ne contenant pas d'agent de coloration d'oxydation ni d'agent oxydant et comprenant au moins un agent protecteur choisi parmi les filtres UV organiques hydrosolubles ou liposolubles dont le log P est inférieur ou égal à 6, puis l'application sur les fibres d'un mélange eau liquide/vapeur d'eau dont la température est comprise entre 40° et 75°C ; lesdits filtres étant choisis parmi les dérivés cinnamiques et les dérivés de benzophénone.

La présente invention a pour objet également l'utilisation dudit procédé pour protéger les fibres kératiniques teintes artificiellement par coloration directe ou par coloration d'oxydation, en particulier les cheveux humains contre l'action des agents atmosphériques et notamment contre l'action de la lumière.

II est bien connu que les cheveux sont sensibilisés ou fragilisés à des degrés divers par l'action des agents atmosphériques et notamment de la lumière. De nombreuses publications divulguent que la lumière naturelle détruit certains aminoacides des cheveux. Ces agressions altérant la fibre capillaire, elles en diminuent les propriétés mécaniques comme la résistance à la traction, la charge à la rupture et l'élasticité, ou leur résistance au gonflement dans un milieu aqueux. Les cheveux sont alors ternes, rêches et cassants.

On sait également que notamment la lumière a tendance à agresser la couleur naturelle des cheveux ainsi que la couleur artificielle des cheveux teints. La couleur des cheveux s'affaiblit peu à peu ou vire vers des nuances peu esthétiques ou indésirables.

L'effet de la lumière est encore plus visible sur les cheveux teints par une coloration artificielle en particulier la coloration d'oxydation ou la coloration directe. Dans ce cas, une exposition à la lumière conduit à une dégradation des colorants présents à la fois dans le cheveu et à sa surface. Il en résulte un affadissement et/ou un virage importants de la couleur des cheveux '

On recherche depuis de nombreuses années, dans l'industrie cosmétique des substances permettant de protéger les cheveux des dégradations engendrées par les agressions atmosphériques, telles que la lumière. En particulier, on recherche des produits protégeant l'intégrité des fibres kératiniques c'est-à-dire leur composition, leur état de surface, leur couleur naturelle ou artificielle, leurs propriétés mécaniques intrinsèques (la résistance à la traction, la charge à la rupture et l'élasticité, ou leur résistance au gonflement dans un milieu aqueux).

Pour lutter contre ces dégradations de la kératine des cheveux, on a déjà proposé d'utiliser des agents de protection comme les filtres UV organiques, des anti-oxydants, des chélatants ou des agents anti-radicaux libres.

On a ainsi proposé certaines substances susceptibles de filtrer les radiations lumineuses, comme l'acide 2-hydroxy-4-méthoxy benzophénone-5-sulfonique ou ses sels (FR-A-2 627 085) ou l'acide 4-(2-oxo-3-bornylidène méthyl) benzène sulfonique ou ses sels (EP-A-329 032) ou encore la lactoferrine (FR-A-2 673 839).

On connaît dans la demande de brevet japonais JP05-043437 des compositions colorantes contenant l'acide 2-hydroxy-4-méthoxy benzophénone-5-sulfonique ou ses sels, un alcool aromatique et un colorant direct acide.

Cependant, les compositions cosmétiques actuelles contenant des agents protecteurs, ne donnent pas complètement satisfaction et en particulier sur les cheveux colorés par des teintures d'oxydation bleues comme celles obtenues avec des couplages comprenant des méta-phénylènediamines.

On a déjà proposé de traiter par un gaz comprenant de la vapeur d'eau des cheveux colorés par oxydation (application d'une composition comprenant au moins un précurseur de coloration d'oxydation et d'une composition comprenant au moins un agent oxydant) afin d'accélérer le processus de coloration dans le brevet FR-1011155 ou pour diminuer la sélectivité des cheveux après coloration dans le brevet EP0659396.
On a déjà proposé dans le brevet FR2713929 de traiter par un gaz comprenant de la vapeur d'eau des cheveux ayant subi une décoloration par application d'une composition contenant un agent oxydant afin d'obtenir une une décoloration rapide qui n'engendre aucun reflet roux.
La demanderesse a maintenant découvert de manière surprenante un nouveau procédé de traitement des fibres kératiniques teintes artificiellement par coloration directe ou par coloration d'oxydation, en particulier des cheveux humains comprenant l'application sur lesdites fibres kératiniques comprenant l'application sur lesdites fibres kératiniques d'une composition ne contenant pas d'agent de coloration d'oxydation ni d'agent oxydant et comprenant au moins un agent protecteur choisi parmi les filtres UV organiques hydrosolubles ou liposolubles dont le log P est inférieur ou égal à 6, puis l'application sur les fibres d'un mélange eau liquide/vapeur d'eau dont la température est comprise entre 40 et 75°C; lesdits filtres étant choisis parmi les dérivés cinnamiques et les dérivés de benzophénone.

Ce procédé permet d'obtenir une meilleure protection desdites fibres contre l'action des agents atmosphériques et notamment contre les effets néfastes de la lumière.

On obtient en particulier grâce à ce procédé de traitement une amélioration de la résistance à la lumière de la coloration des cheveux teints par coloration directe ou coloration par oxydation. Le procédé de traitement selon l'invention permet également d'apporter un effet de protection vis à vis de la lumière durable aux shampooings.
Toutes ces découvertes sont à la base de la présente invention.
Ainsi, selon la présente invention, il est maintenant proposé un procédé de traitement des fibres kératiniques teintes artificiellement par coloration directe ou par coloration d'oxydation, en particulier les cheveux humains, caractérisé en ce qu'il consiste à appliquer sur lesdites fibres kératiniques une composition sans agent de coloration d'oxydation et sans agent oxydant comprenant dans un milieu physilogiquement acceptable au moins un agent protecteur choisi parmi les filtres UV organiques hydrosolubles ou liposolubles dont le log P est inférieur ou égal à 6 ; lesdits filtres étant choisis parmi les dérivés cinnamiques et les dérivés de benzophénone, puis à appliquer sur les fibres un mélange eau liquide/vapeur d'eau dont la température est comprise entre 40 à 75°C.

On entend par « ne contenant pas d'agent de coloration d'oxydation» ne contenant pas de précurseur dé coloration d'oxydation tels que des bases ou des coupleurs dans une quantité suffisante permettant de colorer par oxydation les fibres kératiniques au contact d'un agent oxydant.
On entend par « ne contenant pas d'agent oxydant » par ne contenant pas d'agent oxydant dans une quantité permettant de décolorer par oxydation les fibres kératiniques.
Un autre objet de l'invention consiste en l'utilisation dudit procédé pour la protection des fibres kératiniques contre l'action des agents atmosphériques et notamment contre l'action de la lumière.
Un objet de l'invention consiste en l'utilisation dudit procédé en post traitement d'une coloration d'oxydation ou d'une coloration directe des fibres kératiniques et plus particulièrement des cheveux

Un autre objet de l'invention concerne un procédé de coloration des fibres kératiniques en particulier des cheveux humains comprenant au moins les étapes a), b) et c) suivantes :
a) on effectue une coloration directe ou d'oxydation desdites fibres ;
b) on applique sur lesdites fibres une composition ne contenant pas d'agent de coloration d'oxydation ni d'agent oxydant et comprenant dans un milieu physiologiquement acceptable et en particulier cosmétiquement acceptable, au moins un agent protecteur choisi parmi les filtres UV organiques hydrosolubles ou liposolubles dont le log P est inférieur ou égal à 6 ;
c) on applique sur lesdites fibres un mélange eau liquide/vapeur d'eau dont la température est comprise entre 40 et 75°C ; lesdits filtres étant choisis parmi les dérivés cinnamiques et les dérivés de benzophénone.

Bien que l'exposé qui suit s'articule essentiellement autour du cas particulier du traitement des cheveux, on notera ici que le procédé selon l'invention est applicable à toute fibre kératinique humaine en général, notamment cils, moustaches, poils et autres.

Les différents objets de l'invention vont maintenant être détaillés. L'ensemble des significations et définitions des composés utilisés dans la présente invention données ci-dessous sont valables pour l'ensemble des objets de l'invention.

Dans le cadre de l'invention, la valeur du log P représente de façon classique le coefficient de partage du colorant entre l'octanol et l'eau. La valeur du log P peut être calculée selon la méthode décrite dans l'article de Meylan et Howard « Atom / Fragment contribution method for estimating octanol-water partition coefficient », J. Pharm. Sci. 84 :83-92, 1995. Cette valeur peut aussi être calculée à partir de nombreux logiciels disponibles sur le marché qui détermine la valeur de logP en fonction de la structure d'une molécule. A titre d'exemple, on peut citer le logiciel Epiwin de l'agence de l'environnement des Etats-Unis ainsi que le logiciel Virtual Computational Chemistry Laboratory.

Le mélange eau liquide/vapeur d'eau constitue une brume. Ledit mélange peut contenir en plus au moins un autre gaz tel que l'oxygène ou l'azote, des mélanges de gaz tels que l'air ou bien encore d'autres composés vaporisables.

De préférence, le mélange eau liquide/vapeur d'eau est mis en contact avec la fibre pendant une durée allant de 1 seconde à 1 heure, et encore plus préférentiellement de 5 minutes à 15 minutes. Bien entendu, l'application dudit mélange peut être répétée plusieurs fois sur la même fibre, chaque opération se faisant selon une durée telle qu'indiquée ci-dessus.

Un mode préféré de réalisation du procédé selon l'invention consiste à appliquer tout d'abord sur les cheveux une composition contenant les agents photoprotecteurs, puis à soumettre ces mèches ainsi imprégnées à l'action du mélange eau liquide/vapeur d'eau selon les conditions mentionnées précédemment, puis à refroidir, les mèches ainsi traitées par exemple en envoyant sur ou à travers celles-ci un courant d'air froid ou d'air à température ambiante.
La production du mélange eau liquide/vapeur d'eau utilisé selon l'invention peut se faire à l'aide de tout appareil connu en soi et prévu à cet effet. Toutefois, selon la présente invention, on utilise de préférence un appareil comprenant au moins un générateur de vapeur d'eau directement relié à un casque diffusant sur les fibres kératiniques en particulier les cheveux humains le mélange eau liquide/vapeur d'eau. Comme type d'appareil, on utilisera plus particulièrement celui vendu sous le nom ®MICROMIST par la Société TAKARA BELMONT.

Comme exemples de filtres UV organiques hydrosolubles ou liposolubles dont le log P est inférieur ou égal à 6, on peut citer ceux désignés ci-dessous sous leur nom INCI :
Dérivés cinnamiques :
   Ethylhexyl Methoxycinnamate vendu notamment sous le nom commercial « PARSOL MCX » par HOFFMANN LA ROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   Diisopropyl Methylcinnamate,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF,
   Benzophenone-5
   Benzophenone-6 vendu sous le nom commercial « Helisorb 11 » par Norquay Benzophenone-8 vendu sous le nom commercial « Spectra-Sorb UV-24 » par American Cyanamid
   Benzophenone-9 vendu sous le nom commercial« UVINUL DS-49» par BASF,

Comme filtres UV organiques liposolubles (ou lipophiles) convenant à une mise en oeuvre dans la présente invention, on peut citer plus particulièrement :
Ethylhexyl Methoxycinnamate
Benzophenone-3,

Selon l'invention, le ou les agents protecteurs des fibres kératiniques seront de préférence présents à des concentrations allant de 0,05 % à 50 % en poids, de préférence de 0,35 % à 30% en poids et plus particulièrement de 0,5 à 20% en poids par rapport au poids total de la composition.

Selon une forme préférée de l'invention, on utilisera des agents protecteurs ayant un logP (coefficient de partage octanol/eau) inférieur à 4,5 et plus préférentiellement inférieur à 2.

Selon une forme particulièrement préférée de l'invention, on utilisera des agents protecteurs solubles dans le milieu aqueux de la composition, en particulier des agents protecteurs solubles à 25°C à au moins 0,5% dans l'eau
ou les alcools inférieurs en C₁-C₄ comme l'éthanol.

Selon une forme particulièrement préférée de l'invention, on utilisera des agents protecteurs possédant dans leur structure chimique au moins une fonction acide sous forme libre ou partiellement ou totalement salifiée telle que acide carboxylique ou acide sulfonique. On utilisera encore plus particulièrement des agents protecteurs possédant dans leur structure chimique au moins une fonction acide sulfonique sous forme libre ou partiellement ou totalement salifiée.

Et plus particulièrement, on utilisera des filtres UV organiques hydrosolubles choisis parmi
Benzophenone-4
Benzophenone-5
Benzophenone-9
ou leurs mélanges

Parmi ces filtres on utilisera plus particulièrement la Benzophénone-4.

Le milieu physiologiquement et en particulier cosmétiquement acceptable est de préférence constitué par de l'eau ou par un mélange d'eau et d'au moins un solvant organique cosmétiquement acceptable. A titre de solvant organique, on peut par exemple citer les alcanols inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol et leurs mélanges.

Les solvants sont, de préférence, présents dans des proportions de préférence comprises entre 1 et 40 % en poids environ par rapport au poids total de la composition tinctoriale, et encore plus préférentiellement entre 3 et 30 % en poids environ.

La composition selon l'invention contenant l'agent ou les agents protecteurs peut également renfermer divers adjuvants utilisés classiquement dans les compositions de traitement capillaire, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Selon une forme préférée de l'invention, les compositions capillaires selon l'invention contenant l'agent ou les agents protecteurs comprennent en plus au moins un alcool aromatique et au moins un acide aromatique carboxylique.

Par l'expression "alcool aromatique", on entend tout composé liquide à température ambiante et pression atmosphérique comprenant au moins un cycle benzénique ou naphtalénique et au moins une fonction alcool (OH) directement liée au cycle ou liée sur au moins un substituant dudit cycle. De préférence, la fonction alcool sera sur un substituant du cycle benzénique ou naphtalénique.

Parmi les alcools aromatiques utilisables dans la composition selon l'invention, on peut citer en particulier
- l'alcool benzylique
- le benzoyl isopropanol
- le benzylglycol
- le phénoxyéthanol
- l'alcool di-chloro-benzylique
- le méthylphenylbutanol
- le phénoxyisopropanol
- le phénylisohexanol
- le phénylpropanol
- l'alcool phényléthylique
- leurs mélanges.

On choisira plus particulièrement l'alcool benzylique.

Selon l'invention, le ou les alcools aromatiques peuvent représenter de 0,01 % à 50 % en poids, de préférence de 0,1 % à 30% en poids et plus particulièrement de 1 à 20% en poids par rapport au poids total de la composition. De préférence, ils seront utilisés à des concentrations supérieures à 1% en poids.

Les compositions capillaires selon l'invention comprennent également au moins un acide carboxylique aromatique éventuellement salifié.

Par l'expression "acide carboxylique aromatique", on entend tout composé comprenant au moins un cycle benzènique ou naphtalénique et au moins une fonction acide carboxylique (COOH), sous forme libre ou salifiée, directement liée au cycle ou liée sur au moins un substituant dudit cycle. De préférence, la fonction acide sera directement reliée au cycle benzènique ou naphtalénique.

Les sels des acides carboxyliques aromatiques peuvent être choisis notamment parmi les sels de métal alcalin (sodium, potassium), de métal alcalino-terreux (calcium, magnésium) ou les sels d'amines organiques ou d'ammonium.

Parmi les acides carboxyliques aromatiques utilisables dans la composition selon l'invention, on peut citer en particulier
- l'acide benzoïque
- l'acide para-anisique
- l'acide diphénolique
- l'acide férulique
- l'acide hippurique
- l'acide 3-hydroxybenzoique
- l'acide 4-hydroxybenzoique
- l'acide phénylthioglycolique
- l'acide acétylsalicylique
- l'acide para, méta ou ortho-phtalique ainsi que leurs formes salifiées et leurs mélanges.

On choisira plus particulièrement l'acide benzoïque.

Selon l'invention, le ou les acides aromatiques ou leurs sels peuvent représenter de 0,001 % à 30 % en poids, de préférence de 0,01 % à 20% en poids et plus particulièrement de 0,1 à 10% en poids par rapport au poids total de la composition.

Les compositions selon l'invention peuvent contenir en plus un ou plusieurs agents de conditionnement.

Dans le cadre de la présente invention, on entend par « agent conditionneur » tout agent ayant pour fonction l'amélioration des propriétés cosmétiques des cheveux, en particulier la douceur, le démêlage, le toucher, le lissage, l'électricité statique.

Les agents de conditionnement peuvent se présenter sous forme liquide, semi-solide ou solide tels que par exemple des huiles, des cires ou des gommes.

Selon l'invention, les agents conditionneurs peuvent être choisis parmi les huiles de synthèses telles que les poly-oléfines, les huiles végétales, les huiles fluorées ou perfluorées, les cires naturelles ou synthétiques, les silicones, les polymères cationiques non polysaccharides, les composés de type céramide, les tensioactifs cationiques, les amines grasses, les acides gras et leurs dérivés ainsi que les mélanges de ces différents composés.

Les huiles de synthèse sont notamment les polyoléfines en particulier les poly-α-oléfines et plus particulièrement :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
   A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba ;

On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les agents conditionneurs préférés selon l'invention sont les polymères cationiques et les silicones.

Les polymères cationiques non saccharidiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Par polymères non saccharides, on entend les polymères ne contenant pas de liaison glycoside entre des monosaccharides.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 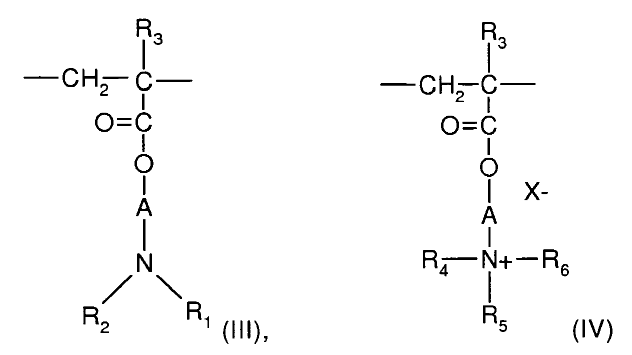 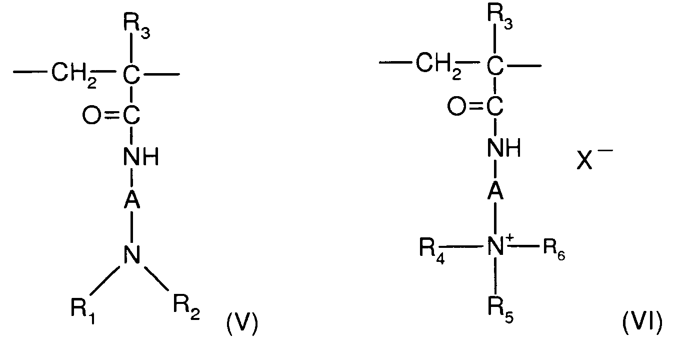 dans lesquelles:
   R₁ et R₂ , identiques ou différents, représentent hydrogène ou un groupe alkyle ayant de 1 à 6 atomes de carbone et de préférence méthyle ou éthyle;
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
      X désigne un anion dérivé d'un acide minéral ou organique tel que un anion méthosulfate ou un halogénure tel que chlorure ou bromure.
   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
   - les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyl-triméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylamino-propyle quaternisé tel que le produit vendu sous la dénomination "GAFQUAT HS 100" par la société ISP.
(2) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(3) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(4) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(5) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(6) les cyclopolymères d'alkyl diallyl amine ou de dialkyl diallyl ammonium tels que les homopolymères ou copolymères comportant comme constituant principal de la chaîne des motifs répondant aux formules (VII) ou (VIII) : 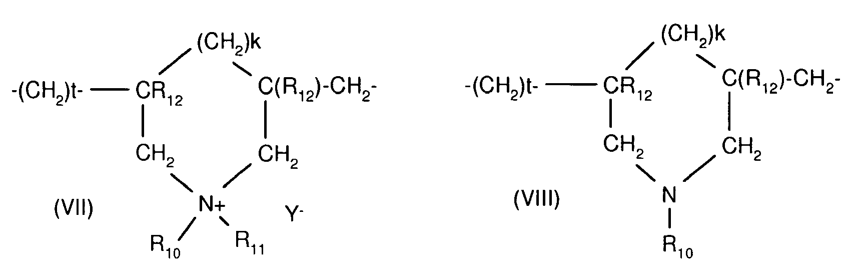 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société NALCO (et ses homologues de faibles masses moléculaires moyenne en poids) et les copolymères de chlorure de diallyldiméthylammonium et d'acrylamide commercialisés sous la dénomination "MERQUAT 550".
(7) le polymère de diammonium quaternaire contenant des motifs récurrents répondant à la formule : 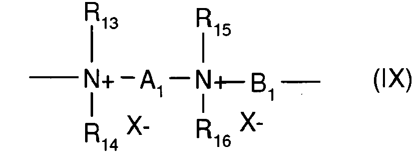 formule (IX) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₃ et R₁₅ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH₂)ₙ-CO-D-OC-(CH₂)n- n est un nombre entier variant de 2 à 20 environ
      dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)ₓ -CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- ;
      De préférence, X⁻ est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
      On peut utiliser plus particulièrement les polymères qui sont constitués de motifs récurrents répondant à la formule : 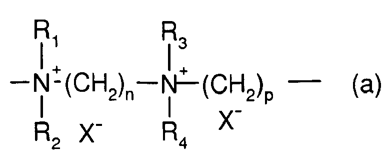 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
      Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCl (CTFA).
(8) les polymères de polyammonium quaternaires constitués de motifs de formule (X): 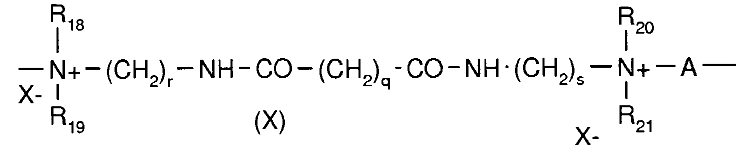 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogènure,
   A désigne un radical d'un dihalogénure ou représente de préférence -CH₂-CH₂-O-CH₂-CH₂-.
      De tels composés sont notamment décrits dans la demande de brevet EP-A-122324.
      On peut par exemple citer parmi ceux-ci, les produits "Mirapol® A 15", "Mirapol® AD1", "Mirapol® AZ1" et "Mirapol® 175" vendus par la société Miranol.
(9) Les polymères quaternaires de vinylpyrrolidone et de vinylimidazole tels que par exemple les produits commercialisés sous les dénominations Luviquat® FC 905, FC 550 et FC 370 par la société B.A.S.F.
(10) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE^{®} SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole et leurs mélanges.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 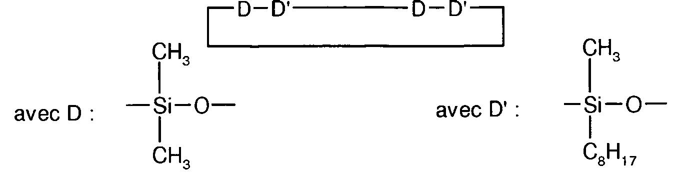 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE .

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHODIA CHIMIE ;
- les huiles des séries RHODORSIL 70 633 et 763 de RHODIA CHIMIE ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.
   Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (XI) : 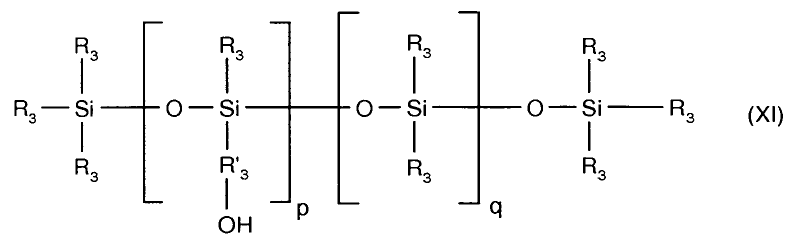 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
   - des groupements acyloxyalkyle tels que par exemple les polyorganosiloxanes décrits dans le brevet US-A-4957732 et répondant à la formule (XII) : 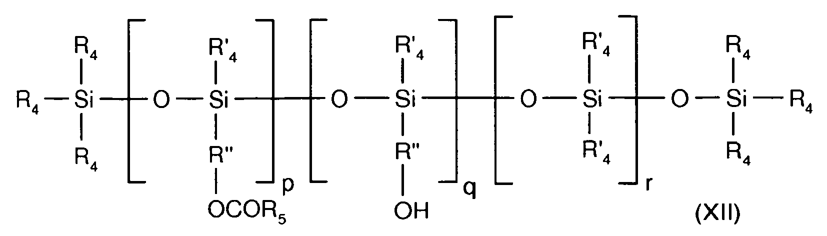 dans laquelle :
      R₄ désigne un groupement méthyle, phényle, -OCOR₅, hydroxyle, un seul des radicaux R₄ par atome de silicium pouvant être OH ;
      R'₄ désigne méthyle, phényle ; au moins 60 % en proportion molaire de l'ensemble des radicaux R₄ et R'₄ désignant méthyle ;
      R₅ désigne alkyle ou alcényle en C₈-C₂₀ ;
      R" désigne un radical alkylène hydrocarboné divalent, linéaire ou ramifié en C₂-C₁₈ ;
      r est compris entre 1 et 120 inclus ;
      p est compris entre 1 et 30 ;
      q est égal à 0 ou est inférieur à 0,5 p, p + q étant compris entre 1 et 30 ; les polyorganosiloxanes de formule (XII) peuvent contenir des groupements : 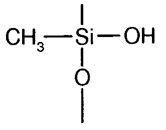 dans des proportions ne dépassant pas 15 % de la somme p + q + r.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 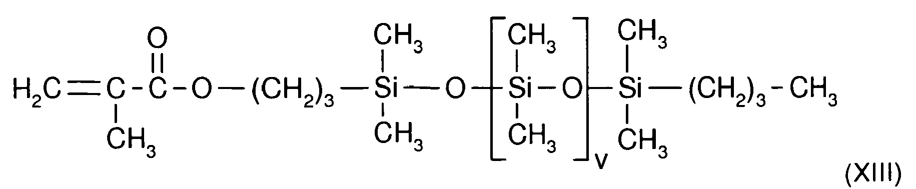 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Les protéines ou hydrolysats de protéines cationiques sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société MAYBROOOK et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "Croquat L" dont les groupements ammonium quaternaires comportent un groupement alkyle en C₁₂ ;
- le "Croquat M" dont les groupements ammonium quaternairees comportent des groupements alkyle en C₁₀-C₁₈ ;
- le "Croquat S" dont les groupements ammonium quaternaires comportent un groupement alkyle en C₁₈ ;
- le "Crotein Q" dont les groupements ammonium quaternaires comportent au moins un groupe alkyle ayant de 1 à 18 atomes de carbone.
Ces différents produits sont vendus par la Société Croda.

D'autre protéines ou hydrolysats quaternisés sont par exemple ceux répondant à la formule (XIV) : 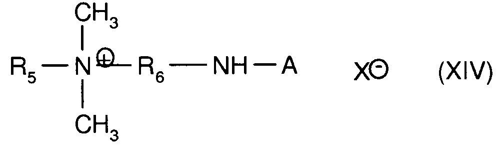 dans laquelle X⁻ est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Selon la présente invention, Les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique
- le N-docosanoyl N-méthyl-D-glucamine
ou les mélanges de ces composés.

On peut également utiliser des tensioactifs cationiques parmi lesquels on peut citer en particulier : les sels d'amines grasses primaires, secondaires ou tertiaires, éventuellement polyoxyalkylénées ; les sels d'ammonium quaternaire ; les dérivés d'imidazoline ; ou les oxydes d'amines à caractère cationique.

Les sels d'ammonium quaternaires sont par exemple :
- ceux qui présentent la formule générale (XV) suivante : 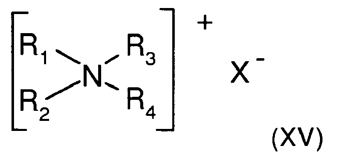
dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (XVI) suivante : 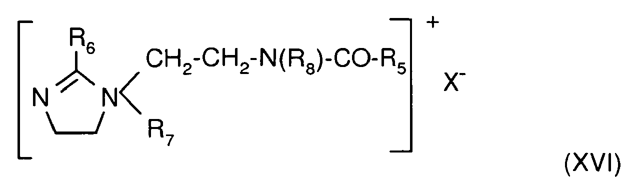
dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société DEGUSSA,
- les sels de diammonium quaternaire de formule (XVII) : 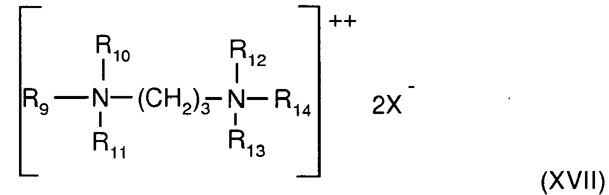
dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester

Les sels d'ammonium quaternaire contenant au moins une fonction ester utilisables selon l'invention sont par exemple ceux de formule (XVIII) suivante 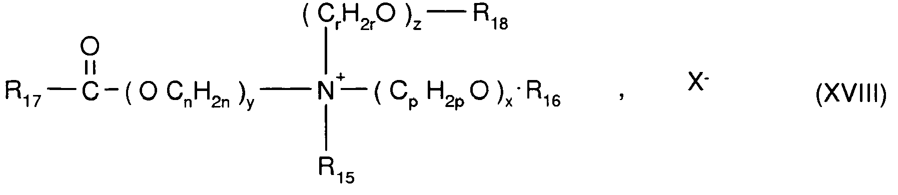 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
   - le radical 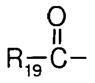
   - les radicaux R₂₀ hydrocarbonés en C₁-C₂₂ linéaires ou ramifiés, saturés
   ou insaturés,
   - l'atome d'hydrogène,
- R₁₈ est choisi parmi :
   - le radical 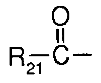
   - les radicaux R₂₂ hydrocarbonés en C₁-C₆ linéaires ou ramifiés, saturés
   ou insaturés,
   - l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés
ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement les sels d'ammonium de formule (XVIII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 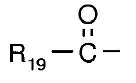
- les radicaux méthyle, éthyle ou hydrocarbonés en C₁₄-C₂₂
   - l'atome d'hydrogène ;
- R₁₈ est choisi parmi :
   - le radical 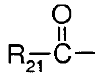
   - l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.
Avantageusement, les radicaux hydrocarbonés sont linéaires.

On peut citer par exemple les composés de formule (XVI) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société COGNIS, STEPANQUAT par la société STEPAN, NOXAMIUM par la société CECA, REWOQUAT WE 18 par la société DEGUSSA.

On peut aussi utiliser les sels d'ammonium contenant au moins une fonction ester décrits dans les brevets US-A-4874554 et US-A-4137180.

Parmi les sels d'ammonium quaternaire de formule (XV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Les acides gras sont choisis plus particulièrement parmi l'acide myristique, l'acide palmitique, l'acide stéarique, l'acide béhénique, l'acide oléïque, l'acide linoléïque, l'acide linolénique et l'acide isostéarique,

Les dérivés d'acides gras sont notamment les esters d'acides carboxyliques en particulier les esters mono, di, tri ou tétracarboxyliques.
Les esters d'acides monocarboxyliques sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle, le néopentanoate d'isostéaryle, le néopentanoate d'isodécyle.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.
On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle, le néopentanoate d'isostéaryle, le néopentanoate d'isodécyle.

Les huiles fluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers ;

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE FO par la société NIPPON OIL.

Il est bien entendu possible de mettre en oeuvre des mélanges d'agents conditionneur.

Selon l'invention, le ou les agents conditionneurs peuvent représenter de 0,001 % à 20 % en poids, de préférence de 0,01 % à 10% en poids et plus particulièrement de 0,1 à 3% en poids par rapport au poids total de la composition finale.

Les compositions selon l'invention peuvent se présenter sous forme de lotions aqueuses ou hydroalcooliques pour le soin des cheveux. Les compositions cosmétiques selon l'invention peuvent également se présenter sous forme de gel, de lait, de crème, d'émulsion ou de mousse et être utilisées les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Le pH de la composition appliquée sur les fibres kératiniques varie généralement de 1 à 11. II est de préférence de 2 à 6, et peut être ajusté à la valeur désirée au moyen d'agents acidifiants ou alcalinisants bien connus de l'état de la technique des compositions appliquées sur des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule suivante : 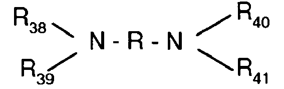 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Selon un mode particulier de réalisation, le procédé de traitement est appliqué sur des fibres kératiniques en particulier des cheveux teints par coloration directe ou par coloration d'oxydation.

Un autre objet de l'invention consiste en l'utilisation dudit procédé pour la protection des fibres kératiniques contre l'action des agents atmosphériques et notamment contre l'action de la lumière.

Un objet de l'invention consiste en l'utilisation dudit procédé en post traitement d'une coloration d'oxydation ou d'une coloration directe des fibres kératiniques et plus particulièrement des cheveux

Un autre objet de l'invention concerne un procédé de coloration des fibres kératiniques en particulier des cheveux humains comprenant au moins les étapes a), b) et c) suivantes :
a) on effectue une coloration directe ou d'oxydation desdites fibres
b) on applique sur lesdites fibres une composition comprenant dans un milieu physiologiquement acceptable et en particulier cosmétiquement acceptable, au moins un agent protecteur choisi parmi les filtres UV organiques hydrosolubles ou liposolubles dont le log P est inférieur ou égal à 6
c) on applique sur lesdites fibres un mélange eau liquide/vapeur d'eau dont la température est comprise entre 40 et 75°C, lesdits filtres étant choisis parmi les dérivés cinnamiques et les dérivés de benzophénone.

Un mode particulier de procédé de coloration des fibres comprend les étapes suivantes :
1) on applique sur les dites fibres a) une composition (A) colorante directe ou d'oxydation pendant un temps suffisant pour développer la couleur,
2) éventuellement, on effectue ensuite un rinçage et/ou un lavage au shampoing et/ou un séchage partiel ou total desdites fibres ,
3) on applique une composition (B) comprenant au moins un agent protecteur telle que définie précédemment et ne contenant pas d'agent de coloration d'oxydation ni d'agent oxydant ;
4) éventuellement, on effectue ensuite un rinçage et/ou un lavage au shampoing et/ou un séchage partiel ou total desdites fibres,
5) on applique sur les fibres un mélange eau liquide/vapeur d'eau dont la température est comprise entre 40 et 75°C;
6) éventuellement, on effectue ensuite un rinçage et/ou un lavage au shampoing et/ou un séchage partiel ou total desdites fibres,

Un deuxième mode particulier de procédé de coloration des fibres comprend les étapes suivantes :
1) on applique une composition (B) comprenant au moins un agent protecteur telle que définie précédemment et ne contenant pas d'agent de coloration d'oxydation ni d'agent oxydant ;
2) on effectue éventuellement un rinçage et/ou un lavage au shampoing et/ou un séchage partiel ou total desdites fibres ,
3) on applique sur les fibres un mélange eau liquide/vapeur d'eau dont la température est comprise entre 40 et 75°C ;
4) on applique sur les dites fibres a) une composition (A) colorante directe ou d'oxydation pendant un temps suffisant pour développer la couleur,
5) on effectue éventuellement un rinçage et/ou un lavage au shampoing et/ou un séchage partiel ou total desdites fibres.
6) éventuellement, on effectue ensuite un rinçage et/ou un lavage au shampoing et/ou un séchage partiel ou total desdites fibres,

Dans les différents modes de procédés de coloration , la composition (B) comprenant le ou les agents protecteurs peut être appliquée immédiatement après coloration ou de manière différée. Par différée, on entend une application se faisant quelques heures, un jour ou plusieurs jours (de 1 à 60 jours) après la coloration. De préférence, la composition (B) sera appliquée immédiatement après coloration des fibres kératiniques.

La nature et la concentration des colorants présents dans la composition (A) colorante n'est pas critique.

Dans le cas des colorations directes éclaircissantes les compositions colorantes (A) résultent du mélange au moment de l'emploi d'une composition colorante (A1) contenant au moins un colorant direct et d'une composition (A2) contenant un agent oxydant.

Dans le cas des colorations d'oxydation, les compositions colorantes (A) résultent du mélange au moment de l'emploi d'une composition colorante (A1) contenant au moins une base d'oxydation et éventuellement au moins un coupleur et/ou un colorant direct et d'une composition (A2) contenant un agent antioxydant.

Les colorants directs sont plus particulièrement des composés absorbant les radiations lumineuses dans le domaine visible (400-750 nm). Ils peuvent être de nature non ionique, anionique ou cationique.

Généralement, les colorants directs sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ceux dérivés du triarylméthane, seuls ou en mélanges.

Parmi les colorants benzéniques nitrés, on peut citer les composés rouges ou orangés suivants : le 1-hydroxy-3-nitro-4-N-(γ-hydroxypropyl)amino benzène, le N-(β-hydroxy éthyl)amino-3-nitro-4-amino benzène, le 1-amino-3-méthyl-4-N-(β-hydroxyéthyl)amino-6-nitro benzène, le 1-hydroxy-3-nitro-4-N-(β-hydroxyéthyl)-amino benzène, le 1,4-diamino-2-nitrobenzène, le 1-amino-2-nitro-4-méthylamino benzène, la N-(β-hydroxyéthyl)-2-nitro-paraphénylènediamine, le 1-amino-2-nitro-4-(β-hydroxy éthyl)amino-5-chloro benzène, la 2-nitro-4-amino-diphénylamine, le 1-amino-3-nitro-6-hydroxybenzène, le 1-(β-amino éthyl)amino-2-nitro-4-(β-hydroxy éthyloxy) benzène, le 1-(β, γ-dihydroxypropyl)oxy-3-nitro-4-(β-hydroxyéthyl)amino benzène, le 1-hydroxy-3-nitro-4-aminobenzène, le 1-hydroxy-2-amino-4,6-dinitrobenzène, le 1-méthoxy-3-nitro-4-(β-hydroxyéthyl)amino benzène, la 2-nitro-4'-hydroxydiphénylamine, le 1-amino-2-nitro-4-hydroxy-5-méthylbenzène, seuls ou en mélanges.

En ce qui concerne les colorants directs benzéniques nitrés, on peut mettre en oeuvre des colorants de ce type jaunes et jaune-verts, comme par exemple le 1-β-hydroxyéthyloxy-3-méthylamino-4-nitrobenzène, le 1-méthylamino-2-nitro-5-(β,γ-dihydroxypropyl)oxy benzène, le 1-(β-hydroxyéthyl)amino-2-méthoxy-4-nitrobenzène, le 1-(β-amino éthyl)amino-2-nitro-5-méthoxy-benzène, le 1,3-di(β-hydroxyéthyl)amino-4-nitro-6-chloro benzène, le 1-amino-2-nitro-6-méthyl-benzène, le 1-(β-hydroxyéthyl)amino-2-hydroxy-4-nitrobenzène, la N-(β-hydroxyéthyl)-2-nitro-4-trifluorométhylaniline, l'acide 4-(β-hydroxy éthyl)amino-3-nitro-benzènesulfonique, l'acide 4-éthylamino-3-nitro-benzoïque, le 4-(β-hydroxyéthyl)amino-3-nitro-chlorobenzène, le 4-(β-hydroxyéthyl)amino-3-nitrométhyl benzène, le 4-(β, γ-dihydroxypropyl)amino-3-nitro-trifluorométhylbenzène, le 1-(β-uréido éthyl)amino-4-nitrobenzène, le 1,3-diamino-4-nitrobenzène, le 1-hydroxy-2-amino-5-nitrobenzène, le 1-amino-2-[tris(hydroxyméthyl)méthyl]amino-5-nitro-benzène, le 1-(β-hydroxyéthyl)amino-2-nitrobenzène, le 4-(β-hydroxyéthyl)amino-3-nitrobenzamide.

Il est de même envisageable d'utiliser des colorants benzéniques nitrés bleus ou violets, comme entre autres le 1-(β-hydroxyéthyl)amino-4-N,N-bis-(β-hydroxyéthyl)amino 2-nitrobenzène, le 1-(γ-hydroxypropyl)amino 4-N,N-bis-(β-hydroxyéthyl)amino 2-nitrobenzène, le 1-(β-hydroxyéthyl)amino 4-(N-méthyl, N-β-hydroxyéthyl)amino 2-nitrobenzène, le 1-(β-hydroxyéthyl)amino 4-(N-éthyl, N-β-hydroxyéthyl)amino 2-nitrobenzène, le 1-(β,γ-dihydroxypropyl)amino 4-(N-éthyl, N- β -hydroxyéthyl)amino 2-nitrobenzène, les 2-nitroparaphénylènediamines de formule suivante : 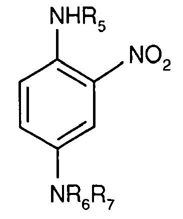 dans laquelle :
R₆ représente un radical alkyle en C₁-C₄, un radical β-hydroxyéthyle ou β-hydroxypropyle ou γ-hydroxypropyle ;
R₅ et R₇, identiques ou différents, représentent un radical β-hydroxyéthyle, β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français FR 2 692 572.

Il est rappelé que les colorants azoïques sont des composés comportant dans leur structure au moins un enchaînement -N=N- non inclus dans un cycle ; les colorants méthiniques sont des composés comportant dans leur structure au moins un enchaînement -C=C- non inclus dans un cycle ; les colorants azométhinique sont des composés comportant dans leur structure au moins un enchaînement-C=N- non inclus dans un cycle.

Les colorants dérivés de triarylméthane comportent dans leur structure au moins un enchaînement suivant : 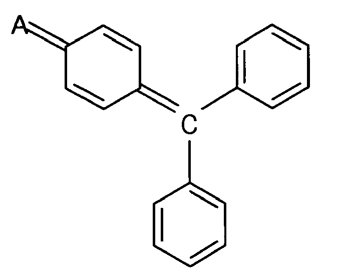 A désignant un atome d'oxygène ou d'azote

Les colorants xanthéniques comportent dans leur structure au moins un enchaînement de formule :

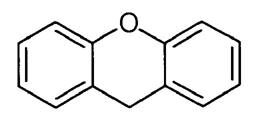

Les colorants phénanthridiniques comportent dans leur structure au moins un enchaînement de formule

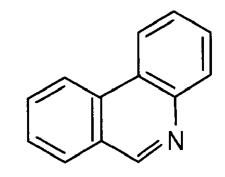

Les colorants phtalocyanines comportent dans leur structure au moins un enchaînement de formule :

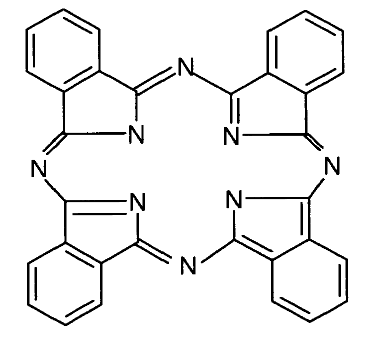

Les colorants phénotiaziniques comportent dans leur structure au moins un enchaînement suivant :

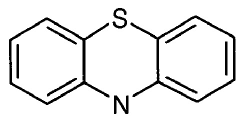

Les colorants directs peuvent de plus être choisis parmi les colorants basiques comme ceux listés dans le Color Index, 3ème édition, notamment sous les dénominations "Basic Brown 16", "Basic Brown 17", "Basic Yellow 57", "Basic Red 76", "Basic Violet 10", "Basic Blue 26" et "Basic Blue 99" ; ou parmi les colorants directs acides, listés le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore les colorants directs cationiques tels que ceux décrits dans les demandes de brevet WO 95/01772, WO 95/15144 et EP 714954 et en particulier « Basic Red 51 », « Basic Orange 31 », « Basic Yellow 87 » dont le contenu fait partie intégrante de la présente invention.

Lorsqu'ils sont présents, le ou les colorants directs représentent de préférence de 0,0005 à 12 % en poids environ du poids total de la composition tinctoriale, et encore plus préférentiellement de 0,005 à 6 % en poids environ de ce poids.

Les bases d'oxydation peuvent être choisies parmi les bases d'oxydation classiquement utilisées en teinture d'oxydation et parmi lesquelles on peut notamment citer les paraphénylènediamines , les bis-phénylalkylènediamines, les paraaminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]-pyrimidin-7-yl)-(2-hydroxy-éthyl)-amino]-éthanol, le 2-[(7-amino-pyrazolo[1,5-a]-pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols, les naphtols et les coupleurs hétérocycliques tels que par exemple les dérivés indoliques, les dérivés indoliniques, les dérivés pyridiniques, les dérivés indazoliques, les dérivés de pyrazolo[1,5-b]-1,2,4-triazole, les dérivés de pyrazolo[3,2-c]-1,2,4-triazole, les dérivés de benzimidazole, les dérivés de benzothiazole, les dérivés de benzoxazole, les dérivés de 1,3-benzodioxole et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 6-hydroxybenzomorpholine, la 3,5 diamino 2,6-diméthoxy pyridine, le 1-N-(βhydroxyéthyl)amino 3,4 méthylènedioxy benzène, le 2,6 bis(βhydroxyéthylamino)toluène, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants. Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La nature de l'agent oxydant utilisé dans la coloration directe éclaircissante (coloration directe avec un agent oxydant) ou dans la coloration d'oxydation n'est pas critique.

L'agent oxydant est de préférence choisi dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Selon un mode particulier de l'invention, on peut utiliser le procédé de l'invention sur des cheveux sensibilisés par des traitements capillaires autres que ceux de l'invention cités précédemment.

L'invention va être maintenant plus complètement illustrée à l'aide des exemples suivants qui ne sauraient être considérés comme la limitant aux modes de réalisation décrits. Dans tout ce qui suit ou ce qui précède, les pourcentages exprimés sont en poids. Dans les exemples, MA signifie matière active.

### EXEMPLES :

### Exemple 1 :

### Etape de coloration :

Au moment de l'emploi, la composition 1 définie ci-après est mélangée avec de l'eau oxygénée (Eau oxygénée l'Oréal professionnel 20 volumes à 6%) poids pour poids.

Le mélange est ensuite appliqué sur des mèches de cheveux 90% blancs permanentées à raison de 10 g de mélange colorant/g de mèche. Le temps de pause est de 15 minutes de chaque côté de la mèche. La coloration est ensuite stoppée par un rinçage à l'eau.

| **Composition 1 colorante** | **Quantités en grammes** |
|---|---|
| Alcool oléique polyglycérolé à 2 moles de glycérol | 4 |
| Alcool oléique polyglycérolé à 4 moles de glycérol (78% M.A) | 5.69 |
| Acide oléique | 3 |
| Amine oléique 2 OE commercialisée sous la dénomination d' ETHOMEEN 012 par la société AKZO | 7 |
| Laurylamino succinamate de diéthylaminopropyle, sel de sodium à 55% M.A. | 3 |
| Alcool oléique | 5 |
| Diéthanolamide d'acide oléique | 12 |
| Propyléne glycol | 3.5 |
| Alcool éthylique | 7 |
| Dipropylène glycol | 0.5 |
| Monométhyléther de propylène glycol | 9 |
| p-phenylenediamine | 6.10⁻⁴ mol |
| 2,4-Diaminophenoxyethanol HCl | 6.10⁻⁴ mol |
| Métabisulfite de sodium en solution aqueuse à 35% M.A | 0.455 |
| Acétate d'ammonium | 0.8 |
| Antioxydant, séquestrant | q.s |
| Parfum, conservateur | q.s |
| Ammoniaque à 20% de NH₃ | 10 |
| Eau déminéralisée | q.s.p. 100 |

### Etapes de traitement protecteur:

On effectue un traitement protecteur sur les mèches colorées en appliquant la composition 2 indiquée ci-dessous à raison de 2g/g de cheveu. On applique alors pendant 10 minutes un mélange eau liquide/vapeur d'eau maintenu à 45°C par l'appareil MICROMIST commercialisé par la société TAKARA BELMONT.

| **Composition 2** | **Quantités en grammes** |
|---|---|
| Alcool benzylique | 4,0g |
| Acide benzoïque | 0,2g |
| Benzophénone-4 | 5,0g |
| Tampon acide citrique /Trisodium citrate/Triéthanolamine | qsp pH=4 |
| Hydroxyethylcellulose | 1,2g |
| Gomme de Xanthane | 0,4g |
| Conservateurs | qsp |
| Eau | qsp 100g |

Les mèches sont ensuite lavées par le shampooing DOP camomille et séchées.

Un panel de 10 personnes évalue les effets du traitement sur les cheveux colorés par rapport à des cheveux colorés n'ayant pas subi de traitement :
1) tenacité de la couleur après lavages au shampoing,
2) tenacité de la couleur après exposition UV/visible,
3) tenacité de la couleur après lavage au shampoing et exposition à la lumière.

### Etapes de ténacité couleur après lavages aux shampooings :

Une partie des mèches traitées subit une épreuve de 6 shampooings DOP camomille successifs comparativement à des mèches colorées non traitées.

### Etapes de ténacité couleur après exposition UV / visible :

Une autre partie des mèches traitées subit une exposition aux UV/Visible comparativement à des mèches colorées non traitées.

Les mèches sont, pour cela, exposées aux UV sur la moitié de leur longueur pendant une période de 18H par un simulateur solaire à lampe Xénon reproduisant un spectre lumineux reproductible et proche de celui du soleil (Suntest XLS commercialisé par la société Atlas). L'autre moitié de la mèche est maquée par du papier cartonné.

### Etapes de ténacité couleur après lavages aux shampooings puis une exposition aux UV/visible

Une autre partie des mèches traitées subit une épreuve de 6 shampooings DOP camomille successifs puis exposition aux UV/Visible dans les conditions décrites précédemment comparativement à des mèches colorées non traitées.

### Résultats :

Les 10 personnes du panel ont montré d'une manière unanime que les mèches colorées ayant subi le traitement protecteur de l'invention présentent par rapport aux mèches colorées non traitées
(1) une meilleure résistance de la couleur d'origine vis-à-vis des shampooings
(2) une meilleure résistance de la couleur d'origine vis-à-vis de la lumière
(3) une meilleure ténacité de la couleur après lavage au shampoing et exposition à la lumière.

## Revendications

1. Procédé de traitement des fibres kératiniques teintes artificiellement par coloration directe ou par coloration d'oxydation, en particulier des cheveux humains, **caractérisé en ce qu'**il consiste à appliquer sur lesdites fibres kératiniques une composition ne contenant pas d'agent de coloration d'oxydation ni d'agent oxydant et comprenant dans un milieu physiologiquement acceptable au moins un argent protecteur choisi parmi les filtres UV organiques hydrosolubles ou liposolubles dont le log P est inférieur ou égal à 6, puis à appliquer sur les fibres d'un mélange eau liquide/vapeur d'eau dont la température est comprise entre 40 et 75°C ; lesdits filtres organiques étant choisis parmi les dérivés cinnamiques et les dérivés de benzophénone.

2. Procédé selon la revendication 1, où le mélange eau liquide/vapeur d'eau contient en plus un ou plusieurs autres gaz ou composés vaporisables.

3. Procédé selon l'une quelconque des revendications 1 ou 2, où le mélange eau liquide/vapeur d'eau est mis en contact avec les fibres pendant une durée allant de 1 seconde à 1 heure.

4. Procédé selon l'une quelconque des revendications 1 à 3, où le mélange eau liquide/vapeur d'eau est mis en contact avec les fibres pendant une durée allant de 5 minutes à 15 minutes.

5. Procédé selon l'une quelconque des revendications 1 à 4, consistant à refroidir les fibres traitées au mélange eau liquide/vapeur d'eau en envoyant sur ou à travers celles-ci un courant d'air froid ou un courant d'air à température ambiante.

6. Procédé selon l'une quelconque des revendications 1 à 5, où les dérivés cinnamiques sont choisis parmi
Ethylhexyl Methoxycinnamate,
Isopropyl Methoxy cinnamate,
Isoamyl Methoxy cinnamate,
Cinoxate,
DEA Methoxycinnamate,
Diisopropyl Methylcinnamate,

7. Procédé selon l'une quelconque des revendications 1 à 5, où les dérivés de benzophénone sont choisis parmi
Benzophenone-1,
Benzophenone-2
Benzophenone-3,
Benzophenone-4,
Benzophenone-5,
Benzophenone-6,
Benzophenone-8,
Benzophenone-9,

8. Procédé selon l'une quelconque des revendications 1 à 7, où les filtres UV organiques liposolubles sont choisis parmi
Ethylhexyl Methoxycinnamate
Benzophenone-3.

9. Procédé selon l'une quelconque des revendications 1 à 8, où le ou les agents protecteurs des fibres kératiniques ont un log P inférieur à 4,5 et plus préférentiellement inférieur à 2.

10. Procédé selon l'une quelconque des revendications 1 à 9, où le ou les agents protecteurs sont solubles à au moins 0,5% dans l'eau ou les alcools inférieurs en C₁-C₄ à 25°C.

11. Procédé selon l'une quelconque des revendications 1 à 10 où le ou les agents protecteurs présentent dans leur structure au moins une fonction acide sous forme libre ou partiellement ou totalement salifiée.

12. Procédé selon la revendication 11, où le ou les agents protecteurs présentent dans leur structure au moins une fonction acide sulfonique sous forme libre ou partiellement ou totalement salifiée.

13. Procédé selon l'une quelconque des revendications 1 à 12 où le ou les agents protecteurs sont des filtres UV organiques hydrosolubles.

14. Procédé selon la revendication 13, où les filtres UV organiques hydrosolubles sont choisis parmi
Benzophenone-4
Benzophenone-5
Benzophenone-9 ou leurs mélanges.

15. Procédé selon la revendication 14, où l'agent protecteur est la Benzophénone-4.

16. Procédé selon l'une quelconque des revendications 1 à 15, où le ou les agents protecteurs des fibres kératiniques représentent de 0;15 % à 50 % en poids, de préférence de 0,35 % à 30% en poids et plus particulièrement de 0,5 à 20% en poids par rapport au poids total de la composition.

17. Procédé selon l'une quelconque des revendications 1 à 16, où le milieu physiologiquement acceptable est constitué par de l'eau ou par un mélange d'eau et d'au moins un solvant organique cosmétiquement acceptable.

18. Procédé selon la revendication 17, où les solvants organiques sont choisis parmi les alcanols inférieurs en C₁-C₄ ; les polyols et éthers de polyols et leurs mélanges.

19. Procédé selon l'une quelconque des revendications 1 à 18, où la composition comprend un ou plusieurs additifs choisis parmi des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents de pénétration, des agents séquestrants, des parfums, des tampon, des agents dispersants, des agents de conditionnement tels que par exemple des silicones volatiles ou non volatiles, modifiées ou non modifiées, des agents filmogènes, des céramides, des agents conservateurs, des agents opacifiants.

20. Procédé selon l'une quelconque des revendications 1 à 19, où la composition comprend au moins un alcool aromatique et au moins un acide aromatique carboxylique.

21. Procédé selon la revendication 20, où le ou les alcools aromatiques représentent de 0,01 % à 50 % en poids, de préférence de 0,1 % à 30% en poids et plus particulièrement de 1 à 20% en poids par rapport au poids total de la composition.

22. Procédé selon la revendication 21, où le ou les alcools aromatiques sont présents à des concentrations supérieures à 1% en poids.

23. Procédé selon l'une quelconque des revendications 20 à 21, où l'alcool aromatique est l'alcool benzylique

24. Procédé selon l'une quelconque des revendications 19 à 23, où le ou les acides aromatiques ou leurs sels représentent de 0,001 % à 30 % en poids, de préférence de 0,01 % à 20% en poids et plus particulièrement de 0,1 à 10% en poids par rapport au poids total de la composition.

25. Procédé selon l'une quelconque des revendications 19 à 24, où l'acide aromatique est l'acide benzoïque.

26. Procédé selon l'une quelconque des revendications 1 à 25, où la composition comprend en plus au moins un agent conditionneur.

27. Procédé selon la revendication 26, où le ou les agents conditionneurs représentent de 0,001 % à 20 % en poids, de préférence de 0,01 % à 10% en poids et plus particulièrement de 0,1 à 3% en poids par rapport au poids total de la composition finale.

28. Procédé selon l'une quelconque des revendications 1 à 27, où la composition se présente sous forme de lotion aqueuse ou hydroalcoolique ; de gel, de lait, de crème, d'émulsion ou de mousse.

29. Procédé selon l'une quelconque des revendications 1 à 28, où la composition est conditionnée sous forme de vaporisateur, de flacon pompe ou dans un récipient aérosol.

30. Procédé selon l'une quelconque des revendications 1 à 29, où le pH de la composition appliquée sur les fibres kératiniques varie généralement de 1 à 11 et de préférence de 2 à 6.

31. Utilisation du procédé tel que défini selon l'une quelconque des revendications 1 à 30 pour la protection des fibres kératiniques en particulier des cheveux humains contre l'action des agents atmosphériques et notamment contre l'action de la lumière.

32. Utilisation du procédé tel que défini selon l'une quelconque des revendications 1 à 30 en post-traitement d'une coloration d'oxydation ou d'une coloration directe des fibres kératiniques et plus particulièrement des cheveux humains.

33. Procédé de coloration des fibres kératiniques en particulier des cheveux humains comprenant au moins les étapes a), b) et c) suivantes :
a) on effectue une coloration directe ou d'oxydation desdites fibres,
b) on applique sur lesdites fibres une composition comprenant au moins un agent protecteur et ne contenant pas d'agent de coloration d'oxydation ni d'agent oxydant telle que définie dans l'une quelconques des revendications précédentes ;
c) on applique sur lesdites fibres on applique sur les fibres un mélange eau liquide/vapeur d'eau tel que défini dans l'une quelconques des revendications précédentes.

34. Procédé de coloration des fibres kératiniques en particulier des cheveux humains, **caractérisé par le fait qu'**il comprend les étapes suivantes
1) on applique sur les dites fibres a) une composition (A) colorante directe ou d'oxydation pendant un temps suffisant pour développer la couleur,
2) éventuellement, on effectue ensuite un rinçage et/ou un lavage au shampoing et/ou un séchage partiel ou total desdites fibres ,
3) on applique une composition (B) comprenant au moins un agent protecteur et ne contenant pas d'agent de coloration d'oxydation ni d'agent oxydant telle que définie dans l'une quelconques des revendications précédentes;
4) éventuellement, on effectue ensuite un rinçage et/ou un lavage au shampoing et/ou un séchage partiel ou total desdites fibres,
5) on applique sur les fibres un mélange eau liquide/vapeur d'eau tel que défini dans l'une quelconques des revendications précédentes ;
6) éventuellement, on effectue ensuite un rinçage et/ou un lavage au shampoing et/ou un séchage partiel ou total desdites fibres.

35. Procédé de coloration des fibres kératiniques en particulier des cheveux humains, **caractérisé par le fait qu'**il comprend les étapes suivantes :
1) on applique une composition (B) comprenant un agent protecteur et ne contenant pas d'agent de coloration d'oxydation ni d'agent oxydant telle que définie dans l'une quelconques des revendications précédentes ;
2) on effectue éventuellement un rinçage et/ou un lavage au shampoing et/ou un séchage partiel ou total desdites fibres,
3) on applique sur les fibres un mélange eau liquide/vapeur d'eau tel que défini dans l'une quelconques des revendications précédentes ;
4) on applique sur les dites fibres a) une composition (A) colorante directe ou d'oxydation pendant un temps suffisant pour développer la couleur,
5) on effectue éventuellement un rinçage et/ou un lavage au shampoing et/ou un séchage partiel ou total desdites fibres.
6) éventuellement, on effectue ensuite un rinçage et/ou un lavage au shampoing et/ou un séchage partiel ou total desdites fibres.

## Claims

1. Process for treating keratin fibres artificially dyed by direct dyeing or by oxidation dyeing, in particular human hair, **characterized in that** it consists in applying to the said keratin fibres a composition not containing any oxidation dye or any oxidizing agent and comprising, in a physiologically acceptable medium, at least one protecting agent chosen from organic watersoluble and liposoluble UV-screening agents with a log P of less than or equal to 6, and then in applying to the fibres a liquid water/steam mixture at a temperature of between 40°C and 75°C; the said organic screening agents being chosen from cinnamic derivatives and benzophenone derivatives.

2. Process according to Claim 1, in which the liquid water/steam mixture also contains one or more other gases or vaporizable compounds.

3. Process according to either of Claims 1 and 2, in which the liquid water/steam mixture is placed in contact with the fibres for a time ranging from 1 second to 1 hour.

4. Process according to any one of Claims 1 to 3, in which the liquid water/steam mixture is placed in contact with the fibres for a time ranging from 5 minutes to 15 minutes.

5. Process according to any one of Claims 1 to 4, which consists in cooling the fibres treated with the liquid water/steam mixture by sending over or through them a stream of cold air or a stream of air at ambient temperature.

6. Process according to any one of Claims 1 to 5, in which the cinnamic derivatives are chosen from:
Ethylhexyl methoxycinnamate,
Isopropyl Methoxy cinnamate,
Isoamyl Methoxy cinnamate,
Cinoxate,
DEA Methoxycinnamate,
Diisopropyl Methylcinnamate.

7. Process according to any one of Claims 1 to 5, in which the benzophenone derivatives are chosen from:
Benzophenone-1,
Benzophenone-2,
Benzophenone-3,
Benzophenone-4,
Benzophenone-5,
Benzophenone-6,
Benzophenone-8,
Benzophenone-9.

8. Process according to any one of Claims 1 to 7, in which the liposoluble organic UV-screening agents are chosen from:
Ethylhexyl methoxycinnamate,
Benzophenone-3.

9. Process according to any one of Claims 1 to 8, in which the keratin fibre protective agent(s) has (have) a log P of less than 4.5 and more preferentially less than 2.

10. Process according to any one of Claims 1 to 9, in which the protective agent(s) is (are) soluble to at least 0.5% in water or C₁-C₄ lower alcohols at 25°C.

11. Process according to any one of Claims 1 to 10, in which the protective agent(s) have in their structure at least one acid function in free or partially or totally salified form.

12. Process according to Claim 11, in which the protective agent(s) have in their structure at least one sulfonic acid function in free or partially or totally salified form.

13. Process according to any one of Claims 1 to 12, in which the protective agent(s) is (are) water-soluble organic UV-screening agents.

14. Process according to Claim 13, in which the water-soluble organic UV-screening agents are chosen from:
Benzophenone-4,
Benzophenone-5,
Benzophenone-9, or mixtures thereof.

15. Process according to Claim 14, in which the protective agent is Benzophenone-4.

16. Process according to any one of Claims 1 to 15, in which the keratin fibre protective agent(s) represent(s) from 0.15% to 50% by weight, preferably from 0.35% to 30% by weight and more particularly from 0.5% to 20% by weight relative to the total weight of the composition.

17. Process according to any one of Claims 1 to 16, in which the physiologically acceptable medium consists of water or of a mixture of water and of at least one cosmetically acceptable organic solvent.

18. Process according to Claim 17, in which the organic solvents are chosen from C₁-C₄ lower alkanols, polyols and polyol ethers, and mixtures thereof.

19. Process according to any one of Claims 1 to 18, in which the composition comprises one or more additives chosen from anionic, cationic, nonionic, amphoteric and zwitterionic surfactants or mixtures thereof, anionic, cationic, nonionic, amphoteric or zwitterionic polymers or mixtures thereof, mineral or organic thickeners, and in particular anionic, cationic, nonionic and amphoteric polymeric associative thickeners, penetrants, sequestrants, fragrances, buffers, dispersants, conditioning agents, for instance volatile or non-volatile, modified or unmodified silicones, film-forming agents, ceramides, preserving agents and opacifiers.

20. Process according to any one of Claims 1 to 19, in which the composition comprises at least one aromatic alcohol and at least one aromatic carboxylic acid.

21. Process according to Claim 20, in which the aromatic alcohol(s) represent(s) from 0.01% to 50% by weight, preferably from 0.1% to 30% by weight and more particularly from 1% to 20% by weight relative to the total weight of the composition.

22. Process according to Claim 21, in which the aromatic alcohol(s) is (are) present in concentrations of greater than 1% by weight.

23. Process according to any one of Claims 20 to 21, in which the aromatic alcohol is benzyl alcohol.

24. Process according to any one of Claims 19 to 23 which the aromatic acid(s) or salts thereof represent(s) from 0.001% to 30% by weight, preferably from 0.01% to 20% by weight and more particularly from 0.1% to 10% by weight relative to the total weight of the composition.

25. Process according to any one of Claims 19 to 24, in which the aromatic acid is benzoic acid.

26. Process according to any one of Claims 1 to 25, in which the composition also comprises at least one conditioning agent.

27. Process according to Claim 26, in which the conditioning agent(s) represent(s) from 0.001% to 20% by weight, preferably from 0.01% to 10% by weight and more particularly from 0.1% to 3% by weight relative to the total weight of the final composition.

28. Process according to any one of Claims 1 to 27, in which the composition is in the form of an aqueous or aqueous-alcoholic lotion, a gel, a milk, a cream, an emulsion or a mousse.

29. Process according to any one of Claims 1 to 28, in which the composition is packaged in the form of a vaporiser, a pump-dispenser bottle or in an aerosol container.

30. Process according to any one of Claims 1 to 29, in which the pH of the composition applied to the keratin fibres generally ranges from 1 to 11 and preferably from 2 to 6.

31. Use of the process as defined according to any one of Claims 1 to 30 for protecting keratin fibres and in particular human hair against the action of atmospheric agents and especially against the action of light.

32. Use of the process as defined according to any one of Claims 1 to 30 as a post-treatment to oxidation dyeing or direct dyeing of keratin fibres and more particularly of human hair.

33. Process for dyeing keratin fibres, in particular human hair, comprising at least steps a), b) and c) below:
a) direct dyeing or oxidation dyeing of the said fibres is performed,
b) a composition comprising at least one protective agent not containing any oxidation dye or any oxidizing agent and as defined in any one of the preceding claims is applied to the said fibres,
c) a liquid water/steam mixture as defined in any one of the preceding claims is applied to the said fibres.

34. Process for dyeing keratin fibres, in particular human hair, **characterized in that** it comprises the following steps:
1) a direct or oxidation dye composition (A) is applied to the said fibres a) for a time that is sufficient to develop the colour,
2) optionally, the said fibres are then rinsed and/or washed with shampoo and/or partially or totally dried,
3) a composition (B) comprising at least one protective agent not containing any oxidation dye or any oxidizing agent and as defined in any one of the preceding claims is applied,
4) optionally, the said fibres are then rinsed and/or washed with shampoo and/or partially or totally dried,
5) a liquid water/steam mixture as defined in any one of the preceding claims is applied to the fibres,
6) optionally, the said fibres are then rinsed and/or washed with shampoo and/or partially or totally dried.

35. Process for dyeing keratin fibres, in particular human hair, **characterized in that** it comprises the following steps:
1) a composition (B) comprising a protective agent not containing any oxidation dye or any oxidizing agent and as defined in any one of the preceding claims is applied,
2) the said fibres are optionally rinsed and/or washed with shampoo and/or partially or totally dried,
3) a liquid water/steam mixture as defined in any one of the preceding claims is applied to the fibres,
4) a direct or oxidation dye composition (A) is applied to the said fibres a) for a time that is sufficient to develop the colour,
5) the said fibres are optionally rinsed and/or washed with shampoo and/or partially or totally dried,
6) optionally, the said fibres are then rinsed and/or washed with shampoo and/or partially or totally dried.

## Patentansprüche

1. Verfahren zur Behandlung von durch eine Direktfärbung oder durch eine oxidative Färbung künstlich gefärbten Keratinfasern und insbesondere menschlichen Haaren, **dadurch gekennzeichnet, dass** es darin besteht, auf die Keratinfasern eine Zusammensetzung aufzubringen, die weder ein Mittel für die oxidative Färbung noch ein Oxidationsmittel aufweist und die in einem physiologisch akzeptablen Medium mindestens ein Schutzmittel enthält, das unter den wasserlöslichen oder fettlöslichen organischen UV-Filtern ausgewählt ist, deren log P kleiner oder gleich 6 ist, und dann auf die Fasern ein Gemisch aus flüssigem Wasser / Wasserdampf aufzubringen, dessen Temperatur im Bereich von 40 bis 75 °C liegt; wobei die organischen Filter unter den Zimtsäurederivaten und den Benzophenonderivaten ausgewählt sind.

2. Verfahren nach Anspruch 1, wobei das Gemisch flüssiges Wasser / Wasserdampf ferner ein oder mehrere weitere Gase oder verdampfbare Verbindungen enthält.

3. Verfahren nach einem der Ansprüche 1 oder 2, wobei das Gemisch flüssiges Wasser / Wasserdampf während einer Zeitspanne, die im Bereich von 1 Sekunde bis 1 Stunde liegt, mit den Fasern in Kontakt gebracht wird.

4. Verfahren nach einem der Ansprüche 1 bis 3, wobei das Gemisch flüssiges Wasser / Wasserdampf während einer Zeitspanne, die im Bereich von 5 bis 15 Minuten liegt, mit den Fasern in Kontakt gebracht wird.

5. Verfahren nach einem der Ansprüche 1 bis 4, das darin besteht, die mit dem Gemisch flüssiges Wasser / Wasserdampf behandelten Fasern abzukühlen, indem auf diese oder durch diese hindurch ein kalter Luftstrom oder ein Luftstrom von Raumtemperatur geleitet wird.

6. Verfahren nach einem der Ansprüche 1 bis 5, wobei die Zimtsäurederivate ausgewählt sind unter:
Ethylhexyl Methoxycinnamate,
Isopropyl Methoxycinnamate,
Isoamyl Methoxycinnamate,
Cinoxate,
DEA Methoxycinnamate,
Diisopropyl Methylcinnamate.

7. Verfahren nach einem der Ansprüche 1 bis 5, wobei die Benzoplzenonderivate ausgewählt sind unter:
Benzophenone-1,
Benzophenone-2,
Benzophenon-3,
Benzophenone-4,
Benzophenone-5,
Benzophenone-6,
Benzophenone-8,
Benzophenone-9.

8. Verfahren nach einem der Ansprüche 1 bis 7, wobei die fettlöslichen organischen UV-Filter ausgewählt sind unter:
Ethylhexyl Methoxycinnamate,
Benzophenone-3.

9. Verfahren nach einem der Ansprüche 1 bis 8, wobei das oder die Schutzmittel für Keratinfasern einen Wert von log P unter 4,5 und insbesondere unter 2 aufweisen.

10. Verfahren nach einem der Ansprüche 1 bis 9, wobei das oder die Schutzmittel bei 25 °C und zumindest 0,5 % in Wasser oder niederen C₁₋₄-Alkoholen löslich sind.

11. Verfahren nach einem der Ansprüche 1 bis 10, wobei das oder die Schutzmittel in ihrer Struktur mindestens eine Säurefunktion in freier Form oder ganz oder teilweise in der Form des Salzes aufweisen.

12. Verfahren nach Anspruch 11, wobei das oder die Schutzmittel in ihrer Struktur mindestens eine Sulfonsäurefunktion in freier Form oder ganz oder teilweise in der Form des Salzes aufweisen.

13. Verfahren nach einem der Ansprüche 1 oder 12, wobei das oder die Schutzmittel wasserlösliche organische UV-Filter sind.

14. Verfahren nach Anspruch 13, wobei der oder die wasserlöslichen organischen UV-Filter ausgewählt sind unter:
Benzophenone-4,
Benzophenone-5,
Benzophenone-9,
oder deren Gemischen.

15. Verfahren nach Anspruch 14, wobei es sich bei dem Schutzmittel um Benzophenone-4 handelt.

16. Verfahren nach einem der Ansprüche 1 bis 15, wobei das oder die Schutzmittel für Keratinfasern 0,15 bis 50 Gew.-%, vorzugsweise 0,35 bis 30 Gew.-% und insbesondere 0,5 bis 20 Gew.-%, bezogen auf das Gesamtgewicht der Zusammensetzung, ausmachen.

17. Verfahren nach einem der Ansprüche 1 bis 16, wobei das physiologisch akzeptable Medium aus Wasser oder einem Gemisch von Wasser und mindestens einem kosmetisch akzeptablen organischen Lösemittel besteht.

18. Verfahren nach Anspruch 17, wobei die organischen Lösemittel unter den niederen C₁₋₄-Alkoholen; Polyolen und Polyolethern und deren Gemischen ausgewählt sind.

19. Verfahren nach einem der Ansprüche 1 bis 18, wobei die Zusammensetzung einen oder mehrere Zusatzstoffe enthält, die unter den anionischen, kationischen, nichtionischen, amphoteren, zwitterionischen grenzflächenaktiven Stoffen oder deren Gemischen, anionischen, kationischen, nichtionischen, amphoteren, zwitterionischen Polymeren oder deren Gemischen, anorganischen oder organischen Verdickungsmitteln und insbesondere anionischen, kationischen, nichtionischen und amphoteren verdickenden assoziativen Polymeren, Penetrationsmitteln, Maskierungsmitteln, Parfums, Puffern, Dispergiermitteln, Konditioniermitteln, wie beispielsweise flüchtigen oder nicht flüchtigen, modifizierten oder nicht modifizierten Siliconen, Filmbildnern, Ceramiden, Konservierungsmitteln und Trübungsmitteln ausgewählt sind.

20. Verfahren nach einem der Ansprüche 1 bis 19, wobei die Zusammensetzung ferner mindestens einen aromatischen Alkohol und mindestens eine aromatische Carbonsäure enthält.

21. Verfahren nach Anspruch 20, wobei der oder die aromatischen Alkohole 0,01 bis 50 Gew.-%, vorzugsweise 0,1 bis 30 Gew.-% und insbesondere 1 bis 20 Gew.-%, bezogen auf das Gesamtgewicht der Zusammensetzung, ausmachen.

22. Verfahren nach Anspruch 21, wobei der oder die aromatischen Alkohole in Konzentrationen über 1 Gew.-% enthalten sind.

23. Verfahren nach einem der Ansprüche 20 bis 21, wobei der aromatische Alkohol der Benzylalkohol ist.

24. Verfahren nach einem der Ansprüche 19 bis 23, wobei die aromatische(n) Säure(n) oder deren Salze 0,001 bis 30 Gew.-%, vorzugsweise 0,01 bis 20 Gew.-% und insbesondere 0,1 bis 10 Gew.-%, bezogen auf das Gesamtgewicht der Zusammensetzung, ausmachen.

25. Verfahren nach einem der Ansprüche 19 bis 24, wobei die aromatische Säure die Benzoesäure ist.

26. Verfahren nach einem der Ansprüche 1 bis 25, wobei die Zusammensetzung ferner mindestens ein Konditioniermittel enthält.

27. Verfahren nach Anspruch 26, wobei das oder die Konditioniermittel 0,001 bis 20 Gew.-%, vorzugsweise 0,01 bis 10 Gew.-% und insbesondere 0,1 bis 3 Gew.-%, bezogen auf das Gesamtgewicht der fertigen Zusammensetzung, ausmachen.

28. Verfahren nach einem der Ansprüche 1 bis 27, wobei die Zusammensetzung als wässrige oder wässrig-alkoholische Lotion; Gel, Milch, Creme, Emulsion oder Schaum vorliegt.

29. Verfahren nach einem der Ansprüche 1 bis 28, wobei die Zusammensetzung in einem Zerstäuber, Pumpflakon oder Aerosolbehälter konfektioniert ist.

30. Verfahren nach einem der Ansprüche 1 bis 29, wobei der pH-Wert der auf die Keratinfasern aufgebrachten Zusammensetzung im Allgemeinen im Bereich von 1 bis 11 und vorzugsweise 2 bis 6 liegt.

31. Verwendung des Verfahrens, wie es in einem der Ansprüche 1 bis 30 definiert ist, um Keratinfasern und insbesondere menschliche Haare gegen die Einwirkung von in der Atmosphäre vorhandenen Agentien und insbesondere gegen die Einwirkung von Licht zu schützen.

32. Verwendung des Verfahrens, wie es in einem der Ansprüche 1 bis 30 definiert ist, für die Nachbehandlung einer oxidativen Färbung oder einer Direktfärbung von Keratinfasern und insbesondere menschlicher Haare.

33. Verfahren zum Färben von Keratinfasern und insbesondere menschlichen Haaren, das zumindest die folgenden Schritte a), b) und c) umfasst:
a) es wird eine direkte Färbung oder eine oxidative Färbung der Keratinfasern durchgeführt;
b) auf die Fasern wird eine Zusammensetzung aufgebracht, die mindestens ein Schutzmittel enthält und die weder ein Mittel für die oxidative Färbung noch ein Oxidationsmittel aufweist, wie sie in einem der vorhergehenden Ansprüche definiert ist;
c) auf die Fasern wird ein Gemisch flüssiges Wasser / Wasserdampf angewandt, wie es in einem der vorhergehenden Ansprüche definiert ist.

34. Verfahren zum Färben von Keratinfasern und insbesondere menschlichen Haaren, **dadurch gekennzeichnet, dass** es zumindest die folgenden Schritte umfasst:
1) auf die Fasern wird a) eine Zusammensetzung (A) für die Direktfärbung oder oxidative Färbung während einer Zeitspanne aufgebracht, die für die Bildung der Farbe ausreichend ist;
2) gegebenenfalls werden die Fasern dann gespült und/oder mit Haarwaschmittel gewaschen und/oder ganz oder teilweise getrocknet;
3) es wird eine Zusammensetzung (B) aufgebracht, die mindestens ein Schutzmittel enthält und die weder ein Mittel für die oxidative Färbung noch ein Oxidationsmittel aufweist, wie sie in einem der vorhergehenden Ansprüche definiert ist;
4) gegebenenfalls werden die Fasern dann gespült und/oder mit Haarwaschmittel gewaschen und/oder ganz oder teilweise getrocknet;
5) auf die Fasern wird ein Gemisch flüssiges Wasser / Wasserdampf angewandt, wie es in einem der vorhergehenden Ansprüche definiert ist;
6) gegebenenfalls werden die Fasern dann gespült und/oder mit Haarwaschmittel gewaschen und/oder ganz oder teilweise getrocknet.

35. Verfahren zum Färben von Keratinfasern und insbesondere menschlichen Haaren, **dadurch gekennzeichnet, dass** es zumindest die folgenden Schritte umfasst:
1) es wird eine Zusammensetzung (B) aufgebracht, die ein Schutzmittel enthält und die weder ein Mittel für die oxidative Färbung noch ein Oxidationsmittel aufweist, wie sie in einem der vorhergehenden Ansprüche definiert ist;
2) gegebenenfalls werden die Fasern dann gespült und/oder mit Haarwaschmittel gewaschen und/ oder ganz oder teilweise getrocknet;
3) auf die Fasern wird ein Gemisch flüssiges Wasser / Wasserdampf aufgebracht, wie es in einem der vorhergehenden Ansprüche definiert ist;
4) auf die Fasern wird a) eine Zusammensetzung (A) für die Direktfärbung oder oxidative Färbung während einer Zeitspanne aufgebracht, die für die Bildung der Farbe ausreichend ist;
5) gegebenenfalls werden die Fasern dann gespült und/oder mit Haarwaschmittel gewaschen und/oder ganz oder teilweise getrocknet;
6) gegebenenfalls werden die Fasern dann gespült und/oder mit Haarwaschmittel gewaschen und/oder ganz oder teilweise getrocknet.
